# EUROPÄISCHE PATENTSCHRIFT

(11) **EP 1 754 507 B1**
(45) Veröffentlichungstag und Bekanntmachung des Hinweises auf die Patenterteilung: **18.01.2012**
(21) Anmeldenummer: 06014988.7
(22) Anmeldetag: 19.07.2006
(51) Int. Cl.: A61N 1/05

(54) **Implantierbare Elektrodenvorrichtung**
Implantable electrode assembly
Ensemble d'électrode implantable

(30) Priorität: 18.08.2005 DE 102005039040
(43) Veröffentlichungstag der Anmeldung: 21.02.2007
(73) Patentinhaber: Biotronik CRM Patent AG, 6341 Baar (CH)
(72) Erfinder: Junge, Agur, 8424 Embrach (CH); Kolberg, Gernot, 12043 Berlin (DE); Stäger, Hans-Gerd, 45549 Sprockvögel (DE)
(74) Vertreter: Lindner-Vogt, Karin L.

(56) Entgegenhaltungen:
- US-A- 4 217 913
- US-B1- 6 819 959

## Beschreibung

Die Erfindung betrifft eine implantierbare Elektrodenvorrichtung und insbesondere eine kardiologische Elektrodenvorrichtung mit den im Oberbegriff des Anspruches 1 angegebenen Merkmalen.

Eine gattungsgemäße Elektrodenanordnung ist aus verschiedenen Druckschriften bekannt, wie beispielsweise der US 2002/0188338 A1, US 2002/0188340 A1 oder US 2002/0193860 A1.

Diese bekannten Vorrichtungen weisen einen lang gestreckten, schlauchartigen Elektrodenkörper auf, an dessen distalem Ende eine helixförmige Einschraubelektrode zwischen einer eingeschobenen Passivstellung innerhalb des Elektrodenkörpers und einer ausgefahrenen, aktiven Fixierstellung verschiebbar ist. Zweck dieser an der Spitze der Elektrodenvorrichtung sitzenden Einschraubelektrode ist ihre stabile und dauerhafte Verankerung in einem für die jeweilige Diagnostik und Therapie geeigneten kardiologischen Gewebe. Um dies zu gewährleisten, wird die helixförmige Einschraubelektrode nach Art eines Korkenziehers in das Gewebe eingeschraubt, was durch eine Rotationsbewegung der die Einschraubelektrode versorgenden Elektrodenzuleitung beim Ausfahren der Einschraubelektrode aus der eingeschobenen Passiv- in die ausgefahrene Fixierstellung realisiert wird.

Die Umsetzung der Rotationsbewegung der Elektrodenzuleitung in eine zusätzliche translatorische Bewegung der Einschraubelektrode zum Ausfahren in die Fixierstellung wird mit Hilfe einer spindelartigen Nockensteuerung hervorgerufen. Bei den Elektrodenvorrichtungen gemäß den oben bezeichneten Druckschriften greift dazu ein am Elektrodenkörper angeordneter Nockenvorsprung direkt in die Wendel der Einschraubelektrode ein. Aufgrund der Positionierung des Nockens muss der ihn lagernde Elektrodenkörper am distalen Ende der Elektrodenvorrichtung relativ starr und stabil ausgelegt sein. Dies verhindert eine flexiblere Ausgestaltung des distalen Endes, was im Hinblick auf einen möglichst reizarmen Implantationsvorgang bei modernen implantierbaren Elektrodenvorrichtungen als vorteilhaft angesehen wird.

Aus den US 6,819,959 und US 2003/0144722 A1 ist in diesem Zusammenhang bereits eine gewisse räumliche Trennung zwischen der Nockensteuerung für die translatorische Bewegung der Einschraubelektrode und der Elektrode selbst bekannt. Dort ist die Einschraubelektrode mit ihrem proximalen Ende an einem Lagerkörper angeordnet, der mit einem Außengewinde spindelartig in einem entsprechenden Innengewinde im Elektrodenkörper verschiebbar geführt ist. Dabei muss allerdings zum Rotationsantrieb des Lagerkörpers ein gesondertes Stylet durch den Elektrodenkörper eingeführt und drehbar mit dem Lagerkörper gekoppelt werden.

Der Erfindung liegt die Aufgabe zugrunde, die Nockensteuerung zum Aus- und Einschieben der Einschraubelektrode so zu verbessern, dass trotz einer einfachen und kostengünstigen Grundkonstruktion das distale Ende des Elektrodenkörpers mit der Einschraubelektrode flexibler ausgestaltet und ein sicheres Betätigen der Einschraubelektrode ermöglicht werden.

Diese Aufgabe wird durch die im Kennzeichnungsteil des Anspruches 1 angegebenen Merkmale gelöst. Basiskonzept ist dabei die Entkopplung der die Einschraubelektrode bildenden Helix von der deren Aus- und Einfahrbewegung hervorrufenden Nockensteuerung, indem dafür eine separate Nockenwendel im Elektrodenkörper vorgesehen ist. Durch diese Nockenwendel verläuft die Elektrodenzuleitung, die in Form einer Leitungswendel ausgebildet ist und welche im Bereich des distalen Endes der Elektrodenleitung in einem Lagerkörper endet, an dem die Einschraubelektrode gekoppelt ist. In diese Nockenwendel greift ein auf dem Leitungswendelabschnitt der Elektrodenzuleitung relativ dazu drehfest gelagerter Nockenkörper ein.

Ungeachtet der Tatsache, ob der Nockenkörper als geschlossener Nockenring mit mindestens einem in die Nockenwendel eingreifenden Nockenvorsprung oder als offener Helixring mit der Nockenwendel entsprechender Steigung ausgestaltet ist, ermöglicht das erfindungsgemäße Konzept die Anbringung des Nockenkörpers noch auf dem als Leitungswendel ausgebildeten Teil der Elektrodenzuleitung. Eine derartige Leitungswendel ist flexibel und kann mit nur einem kurzen Übergang an die Wendel der Einschraubelektrode angekoppelt werden. Damit lässt sich eine hohe Flexibilität der Elektrodenvorrichtung im Bereich des distalen Endes erreichen.

Der angesprochene geschlossene Nockenring oder offene Helixring ist jeweils genauso ein konstruktiv einfaches, vorzugsweise einstückiges und leicht montierbares Teil wie die damit kooperierende Nockenwendel, bei der es sich vorzugsweise um ein entsprechend formgebogenes Drahtteil handelt.

Zum Schutz der Einschraubelektrode und damit verbundene Elektrodenzuleitung gegen ein "Überdrehen" sowohl beim Ein- als auch Ausfahren sind gemäß einer weiteren bevorzugten Ausführungsform Drehanschläge vorgesehen, die die Rotationsbewegung der Elektrodenzuleitung begrenzen. Diese Drehanschläge können durch in die Bewegungsbahn des Nockenkörpers ragende Vorsprünge am Elektrodenkörper oder - noch einfacher - durch die in die Bewegungsbahn des Nockenkörpers eingebogenen Enden der drahtförmigen Nockenwendel gebildet sein.

Je nach Einsatzzweck der erfindungsgemäßen Elektrodenvorrichtung kann deren distales Ende in unterschiedlichen Ausführungsformen, was die Unterbringung der Nockensteuerung anbetrifft, konfiguriert werden. So ist einerseits eine raumsparende Unterbringung in einem Isoliergehäuse radial innerhalb einer vor dem distalen Elektrodenende angeordneten Ringelektrode vorgesehen. Die Nockensteuerung kann auch in einem separaten Kopfgehäuse mit enger Zuordnung zum Lager der Einschraubelektrode angeordnet werden Gemäß weiteren bevorzugten Ausführungsformen der Erfindung soll der Querschnitt der Einschraubelektrode unrund, insbesondere z.B. ellipsenförmig oder rechteckig ausgebildet sein, wobei die radial zur Schraubachse verlaufende Querschnittsdimension größer als die parallel dazu verlaufende ist. Damit wird der Effekt erzielt, dass bei geringerem Verdrängungsvolumen in axialer Richtung größere Kräfte aufgenommen werden können. So lässt sich die Einschraubelektrode dann mit einem geringeren Drehmoment einschrauben und erzielt höhere Haltekräfte. Die vorstehende Konfiguration der Einschraubwendel lässt sich im Übrigen auch losgelöst vom Kerngedanken der im Hauptanspruch angegebenen Erfindung mit Vorteil realisieren.

Zusammenfassend bildet die Erfindung mit ihren bevorzugten Ausführungsformen eine Basis für mannigfache Vorteile, wie eine einfache, kostengünstige Konstruktion durch eine geringe Teileanzahl und unaufwendige Materialien, geringen Polabstand von der Einschraubelektrode zur nachfolgenden Ringelektrode von unter 10 mm, Schutz der Einschraubelektrode vor Beschädigung durch Überdrehen, eine breitbandige Adaptierbarkeit, z. B. zur Realisierung von DEFI-Elektrodenvorrichtungen, einen hochflexiblen Kopfaufbau und die Ermöglichung, ein "Mapping" mit Hilfe der Einschraubelektrode vornehmen zu können.

Weitere Merkmale, Vorteile und Einzelheiten der Erfindung sind der nachfolgenden Beschreibung entnehmbar, in der Ausführungsbeispiele des Erfindungsgegenstandes anhand der beigefügten Zeichnungen näher erläutert werden. Es zeigen:
- Fig. 1: eine schematische Seitenansicht des distalen Endes einer Elektrodenvorrichtung mit Einschraubelektrode und deren No- ckensteuerung,
- Fig. 2: einen Axialschnitt der Nockensteuerung für die Einschraub- elektrode in einer ersten Ausführungsform,
- Fig. 3 und 4: Detailansichten der Nockensteuerung mit der Einschraubelekt- rode in Fixierstellung bzw. Passivstellung,
- Fig. 5: eine vergrößerte Perspektivdarstellung einer Nockenwendel mit darin eingreifenden Nockenkörper,
- Fig. 6: eine Detailseitenansicht einer Nockensteuerung in einer zwei- ten Ausführungsform, und
- Fig. 7: eine teilweise weggebrochene Seitenansicht des distalen En- des einer Elektrodenvorrichtung in einer zweiten Ausführungs- form.

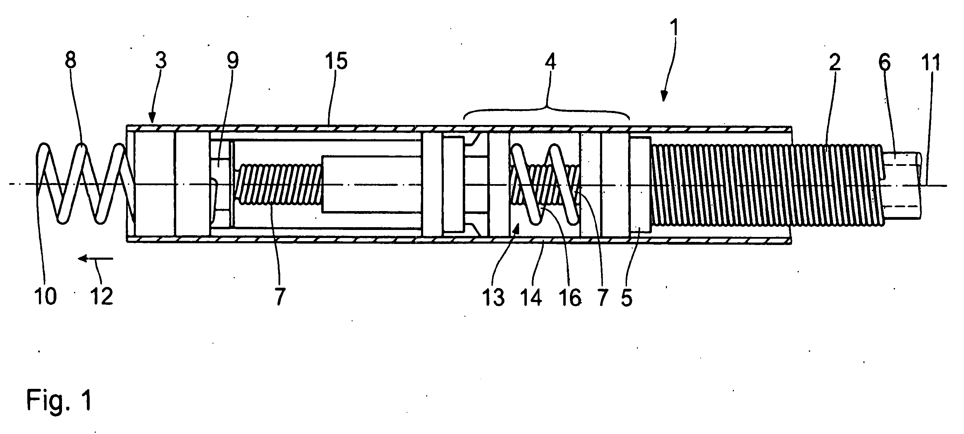

Wie aus Fig. 1 deutlich wird, weist eine kardiologische Elektrodenvorrichtung einen lang gestreckten, schlauchartigen Elektrodenkörper 1 auf, der beispielsweise aus einem flexiblen Silikonmaterial gefertigt ist. Im nicht näher dargestellten Lumen dieses Elektrodenkörpers 1 ist eine erste Leitungswendel 2 großen Durchmessers geführt, die elektrisch mit einer vor dem distalen Ende 3 der Elektrodenvorrichtung angeordneten Ringelektrode 4 über eine Kontakthülse 5 verbunden ist. Innerhalb der äußeren Leitungswendel 2 ist ein Isolierschlauch 6 geführt, in dem wiederum eine weitere Leitungswendel 7 kleineren Durchmessers zur Kontaktierung der am distalen Ende 3 angeordneten Einschraubelektrode 8 läuft. Diese Leitungswendel 7 ist nach Art einer auf Block gewickelten Schraubenfeder aus einem flexiblen Metallmaterial gefertigt und endet im Bereich des distalen Endes 3 in einem Lagerkörper 9 für die Einschraubelektrode 8. Letztere ist als helixförmige Drahtwendel mit einer Spitze 10 ausgebildet und wird zur Platzierung an geeigneter Stelle beispielsweise in der Herzkammer eines Elektrodenempfängers durch eine Rotationsbewegung um die Rotationsachse 11 und eine translatorische Ausfahrbewegung gemäß Pfeil 12 korkenzieherartig in das Herzgewebe eingedreht.

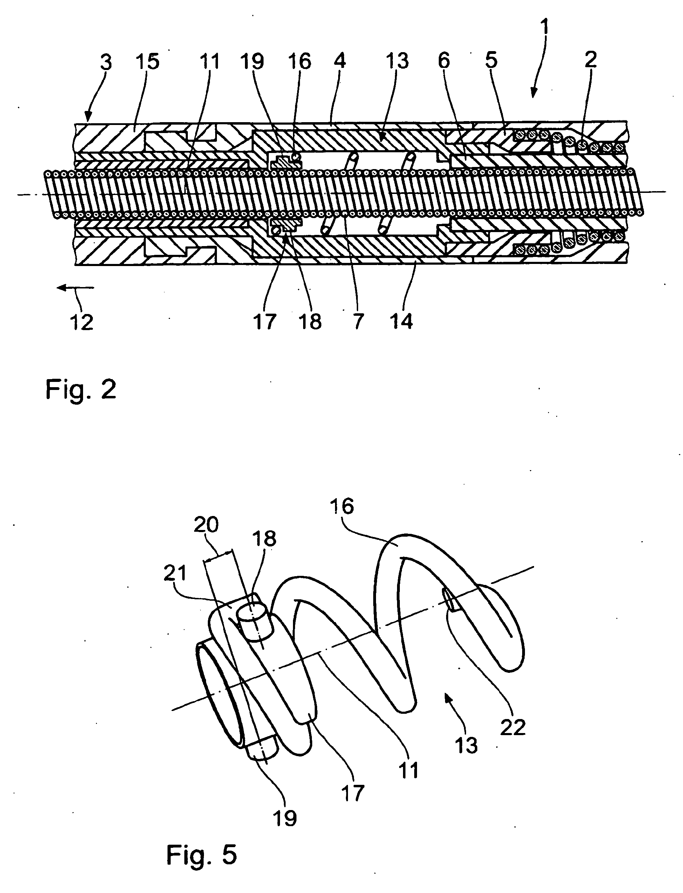

Die Drehbewegung um die Rotationsachse 11 wird durch ein entsprechendes Drehen der Leitungswendel 7 um die eigene Achse vom nicht dargestellten proximalen Ende der Elektrodenvorrichtung aus hervorgerufen. Aus dieser Drehbewegung wird auch die Ausfahrbewegung 12 und gegenläufige Einfahrbewegung mit Hilfe einer als Ganzes mit 13 bezeichneten Nockensteuerung abgeleitet, die im Ausführungsbeispiel gemäß den Fig. 1 und 2 radial innerhalb der Ringelektrode 4 untergebracht ist. Dazu ist letztere auf ihrer Innenseite mit einem aus Isolierwerkstoff - z. B. Silicon - bestehenden Isoliergehäuse 14 versehen, das sich in Richtung distales Ende 3 rohrförmig in eine sich der Ringelektrode 4 anschließende Außenisolierung 15 des Elektrodenkörpers 1 fortsetzt.

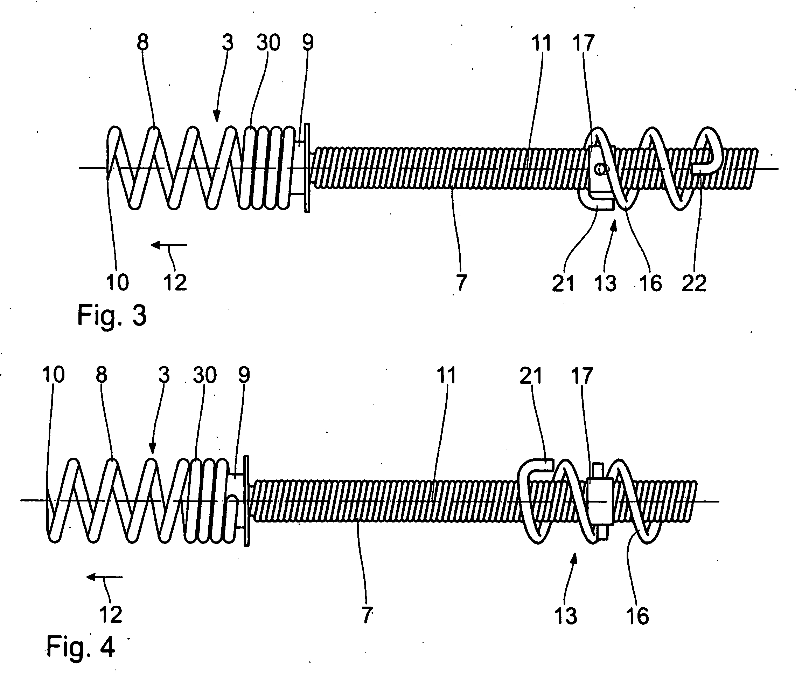

Wie aus den Fig. 2 bis 5 deutlich wird, ist in dem Isoliergehäuse 14 eine Nockenwendel 16 drehfest angeordnet, die aus einem offen gewickelten, formsteifen Draht besteht. Der lichte Innendurchmesser der Nockenwendel 16 ist deutlich größer als der Außendurchmesser der Leitungswendel 7. Auf dieser sitzt im Bereich der Nockensteuerung 13 drehfest ein geschlossener Nockenring 17, der zwei diametral gegenüberliegende, radial abstehende Nockenvorsprünge 18, 19 aufweist. Diese weisen in axialer Richtung einen kleinen Versatz 20 (Fig. 5) auf. Der Außendurchmesser des Nockenrings 7 ist etwas kleiner als der Innendurchmesser der Nockenwendel 16, sodass sich der Ring durch die Nockenwendel 16 in Axialrichtung bewegen kann, wobei die Nockenvorsprünge 18, 19 in die Nockenwendel - wie in den Fig. 3 bis 5 gezeigt - eingreifen. Der einstückig gefertigte Nockenring 17 besteht beispielsweise aus einem Metallmaterial und kann auf die Leitungswendel 7 aufgeschweißt sein.

Bei einer Rotationsbewegung der Leitungswendel 7 wird durch den Eingriff der Nockenvorsprünge 18, 19 in die Nockenwendel 16 der Nockenring 17 in Axialrichtung translatorisch bewegt, sodass die mit der Leitungswendel 7 über den Lagerkörper 9 gekoppelte Einschraubelektrode 8 neben der Rotationsbewegung auch die translatorische Bewegung in Ausfahrrichtung 12 und entgegengesetzt dazu - je nach Drehrichtung der Leitungswendel 7 - erfährt.

Wie aus den Fig. 3 bis 5 deutlich wird, sind die Enden 21, 22 der Nockenwendel 16 parallel zur Rotationsachse 11 nach innen abgebogen, sodass sie in die Bewegungsbahn der Nockenvorsprünge 18, 19 eingreifen und damit als Drehanschläge für die Begrenzung der Rotation der Leitungswendel 7 und damit der Ein- und Ausfahrbewegung der Einschraubelektrode 8 dienen.

Aufgrund der in den Fig. 1 bis 5 gezeigten Anordnungen der Nockensteuerung 13 unter der Ringelektrode 4 kann der Bereich zwischen dieser und dem distalen Ende 3 im Bereich der Außenisolation 15 flexibel gehalten werden, wodurch die Elektrodenvorrichtung in einer sogenannten "Soft-Tip-Ausführung" realisierbar ist.

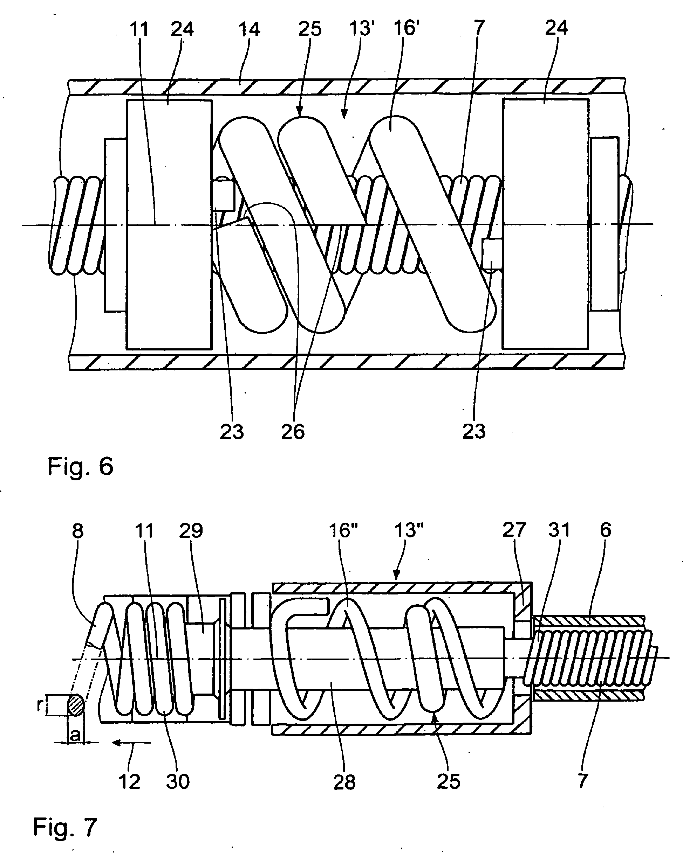

Bei der in Fig. 6 gezeigten Ausführungsform der Nockensteuerung 13' ist wiederum eine drahtförmige Nockenwendel 16 vorgesehen, wobei diese jedoch keine eingebogenen Enden 21, 22 aufweist. Vielmehr sind als Drehanschläge für die Begrenzung der Rotationsbewegung der Leitungswendel 7 jeweils von axial in den Bereich der Nockenwendel 16' eingreifenden Vorsprüngen 21 gebildet, die in axialer Richtung von die Nockensteuerung 13' flankierenden, feststehenden Lagerringen 24 abstehen. Als Nockenkörper ist bei dieser Ausführungsform ferner ein offener Helixring 25 vorgesehen, der analog der Nockenwendel 16' offen gewickelt ist und eine entsprechende Steigung aufweist. Der rotationsfest mit der Leitungswendel 7 verbundene Helixring 25 läuft bei einer Drehung der Leitungswendel 7 in der Nockenwendel 16' koaxial-translatorisch, sodass die Leitungswendel 7 in Ausfahrrichtung 12 bzw. entgegengesetzt dazu - je nach Drehrichtung der Leitungswendel 7 - zum Ein- und Ausfahren der Einschraubelektrode 8 verschoben wird. Die offenen Stirnflächen 26 des Helixringes 25 schlagen zur Begrenzung der Rotations- und Translationsbewegung der Leitungswendel 7 und damit der Einschraubelektrode 8 jeweils am Ende der entsprechenden Vorschubbewegungen an den Vorsprüngen 23 der Lagerringe 24 an.

Bei der in Fig. 7 gezeigten Ausführungsform die nicht Teil der Erfindung bildet, ist die Nockensteuerung 13" unmittelbar vor das distale Ende 3 der Elektrodenvorrichtung gerückt und in einem separaten Kopfgehäuse 27 untergebracht. Dieses dient auch als Lagerung für eine Lagerwelle 28, die mit der Leitungswendel 7 drehfest verbunden ist.

Diese Lagerwelle 28 durchgreift die drehfest im Kopfgehäuse 27 festgelegte Nockenwendel 16" und trägt entsprechend der Ausführungsform gemäß Fig. 6 einen Helixring 25, der mit der Nockenwendel 16" kommt. In Richtung zum distalen Ende 3 ist die Lagerwelle 28 mit einem Lagerkopf 29 versehen, auf dem die - als solche nicht dargestellte - Einschraubelektrode 8 mit Hilfe von eng gewickelten Fixierwindungen 30 befestigt und elektrisch kontaktiert ist. Bei einer Rotation der Leitungswendel 7 wird die Lagerwelle 28 ebenfalls gedreht und in Ausfahrrichtung 12 translatorisch bewegt, sodass die Einschraubelektrode 8 aus dem Elektrodenkörper 1 unter Rotation ausgeschoben und in entsprechendes Gewebe eingeschraubt werden kann. Die Kontaktierung der Einschraubelektrode 8 erfolgt dabei über eine direkte elektrische Verbindung zwischen den Windungen 30, der Lagerwelle 28 und der Leitungswendel 7.

Wie im Übrigen in Fig. 7 angedeutet ist, ist der Querschnitt des Drahtmaterials der Einschraubelektrode 8 ellipsenförmig ausgebildet, wobei die radial zur Schraubachse 11 verlaufende Querschnittsdimension r größer als die parallel dazu verlaufende Querschnittsdimension a ist.

## Patentansprüche

1. Implantierbare Elektrodenvorrichtung, insbesondere kardiologische Elektrodenvorrichtung umfassend
- einen langgestreckten, schlauchartigen Elektrodenkörper (1),
- eine am distalen Ende (3) des Elektrodenkörpers (1) zwischen einer eingeschobenen Passivstellung innerhalb des Elektrodenkörpers (1) und einer ausgefahrenen, aktiven Fixierstellung außerhalb des Elektrodenkörpers (1) verschiebbare, helixförmige Einschraubelektrode (8), die durch eine Rotationsbewegung ihrer Elektrodenzuleitung (7) mit Hilfe einer spindelartigen Nockensteuerung (13, 13', 13") zwischen der eingeschobenen Passiv- und ausgefahrenen Fixierstellung verlagerbar ist, wobei
- die Elektrodenzuleitung (7) einen Leitungswendelabschnitt aufweist, und
- die Nockensteuerung (13, 13', 13") einerseits durch eine separate, im Elektrodenkörper (1) ortsfest und koaxial zur Elektrodenzuleitung (7) gelagerte Nockenwendel (16, 16', 16") und andererseits durch einen auf der Elektrodenzuleitung (7) drehfest dazu gelagerten, in die Nockenwendel (16, 16', 16") eingreifenden Nockenkörper (17, 25) gebildet ist, **dadurch gekennzeichnet, dass** der Nockenkörper auf dem Leitungswendelabschnitt angeordnet ist.

2. Elektrodenvorrichtung nach Anspruch 1, **dadurch gekennzeichnet, dass** der Nockenkörper ein auf dem Leitungswendelabschnitt der Elektrodenzuleitung (7) sitzender, vorzugsweise als einstückiges Formteil ausgebildeter, geschlossener Nockenring (17) ist, der mit mindestens einem Nockenvorsprung (18, 19) in die Nockenwendel (16) eingreift.

3. Elektrodenvorrichtung nach Anspruch 1, **dadurch gekennzeichnet, dass** der Nockenkörper ein auf dem Leitungswendelabschnitt der Elektrodenzuleitung (7) sitzender, offener Helixring (25) mit der Nockenwendel (16', 16") entsprechender Steigung ist, der in die Nockenwendel (16', 16") eingreifend bei einer Rotation der Elektrodenzuleitung (7) koaxialtranslatorisch in der Nockenwendel (16', 16") läuft.

4. Elektrodenvorrichtung nach einem der vorgenannten Ansprüche, **dadurch gekennzeichnet, dass** die Rotationsbewegung der Elektrodenzuleitung (7) durch mit dem Nockenkörper (17, 25) zusammenwirkende, die maximal ausgeschobene Fixierstellung und eingeschobene Passivstellung der Einschraubelektrode (8) definierende Drehanschläge (21, 22; 23) begrenzt ist.

5. Elektrodenvorrichtung nach Anspruch 4, **dadurch gekennzeichnet, dass** die Drehanschläge durch in die Bewegungsbahn des Nockenkörpers (25) ragende Vorsprünge (23) am Elektrodenkörper (1) gebildet sind.

6. Elektrodenvorrichtung nach Anspruch 4, **dadurch gekennzeichnet, dass** die Drehanschläge durch die in die Bewegungsbahn des Nockenkörpers (17) eingebogenen Enden (21, 22) der drahtförmigen Nockenwendel (16, 16") gebildet sind.

7. Elektrodenvorrichtung nach einem der vorgenannten Ansprüche, **dadurch gekennzeichnet, dass** die Nockensteuerung (13, 13') in einem Isoliergehäuse (14) radial innerhalb einer vor dem distalen Elektrodenende (3) angeordneten Ringelektrode (4) untergebracht ist.

8. Elektrodenvorrichtung nach einem der Ansprüche 1 bis 6, **dadurch gekennzeichnet, dass** die Nockensteuerung (13") in einem separaten Kopfgehäuse (27) untergebracht ist.

9. Elektrodenvorrichtung insbesondere nach einem der vorgenannten Ansprüche, **dadurch gekennzeichnet, dass** die Einschraubelektrode (8) aus einem Drahtmaterial mit unrundem Drahtquerschnitt besteht, wobei die radial zur Schraubachse (11) verlaufende Querschnittsdimension (r) größer als die parallel dazu gerichtete Querschnittsdimension (a) ist.

10. Elektrodenvorrichtung nach Anspruch 9, **dadurch gekennzeichnet, dass** der Drahtquerschnitt ellipsenförmig oder rechteckig geformt ist.

## Claims

1. An implantable electrode assembly, and more particularly a cardiac electrode assembly, comprising
- an elongated, tubular electrode body (1),
- a helical screw-in electrode (8), which can be moved at the distal end (3) of the electrode body (1) between an inserted passive position inside the electrode body (1) and an extended, active fixing position outside of the electrode body (1) and which can be displaced by way of a rotational movement of the electrode feed line (7) thereof, with the aid of a spindle-like cam control (13, 13', 13"), between the inserted passive position and the extended fixing position, wherein
- the electrode feed line (7) comprises a coiled line section, and
- the cam control (13, 13', 13") is formed, on the one side, by a separate cam coil (16, 16', 16"), which is mounted in the electrode body (1) in a stationary manner and coaxially with respect to the electrode feed line (7), and, on the other side, by a cam body (17, 25), which is mounted on the electrode feed line (7) non-rotatably with respect to the same and engages with the cam coil (16, 16', 16"), **characterized in that** the cam body is arranged on the coiled line section.

2. The electrode assembly according to claim 1, **characterized in that** the cam body is a closed cam ring (17), which is seated on the coiled line section of the electrode feed line (7) and preferably designed as a single-piece molded part and at least one cam protrusion (18, 19) of which engages in the cam coil (16).

3. The electrode assembly according to claim 1, **characterized in that** the cam body is an open helix ring (25), which is seated on the coiled line section of the electrode feed line (7) and has a pitch that corresponds to that of the cam coil (16, 16") and which runs in the cam coil (16, 16") in a coaxial-translatory manner during a rotation of the electrode feed line (7).

4. An electrode assembly according to any one of the preceding claims, **characterized in that** the rotational movement of the electrode feed line (7) is limited by rotary stops (21, 22; 23) which cooperate with the cam body (17, 25) and define the maximum extended fixing position and inserted passive position of the screw-in electrode (8).

5. The electrode assembly according to claim 4, **characterized in that** the rotary stops are formed by protrusions (23) on the electrode body (1) which project into the movement path of the cam body (25).

6. The electrode assembly according to claim 4, **characterized in that** the rotary stops are formed by the ends (21, 22) of the wire-shaped cam coil (16, 16") which are bent into the movement path of the cam body (17).

7. An electrode assembly according to any one of the preceding claims, **characterized in that** the cam control (13, 13') is accommodated in an insulating housing (14) radially inside an annular electrode (4) that is arranged in front of the distal electrode end (3).

8. An electrode assembly according to any one of claims 1 to 6, **characterized in that** the cam control (13") is accommodated in a separate head housing (27).

9. An electrode assembly, in particular according to any one of the preceding claims, **characterized in that** the screw-in electrode (8) comprises a wire material having a non-circular wire cross-section, wherein the cross-sectional dimension (r) extending radially to the screw axis (11) is greater than the cross-sectional dimension (a) oriented parallel thereto.

10. The electrode assembly according to claim 9, **characterized in that** the wire cross-section has an elliptical or rectangular shape.

## Revendications

1. Dispositif à électrode implantable, en particulier un dispositif à électrode cardiologique, comprenant :
- un corps d'électrode du genre tube (1) allongé en longueur,
- une électrode à vis hélicoïdale (1) déplaçable à l'extrémité distale (3) du corps d'électrode (1) entre une position passive enfoncée dans le corps d'électrode (1) et une position de fixation active sortie à l'extérieur du corps d'électrode (1), apte à être déplacée par un mouvement rotatif de sa sonde (7), à l'aide d'une commande à cames du genre broche (13, 13', 13"), entre la position passive enfoncée et la position de fixation sortie, dans lequel
- la sonde (7) comporte une section de sonde en boudin, et
- la commande à cames (13, 13', 13") est formée d'une part par un boudin à cames (16, 16', 16") particulier, stationnaire et monté de façon coaxiale par rapport à la sonde (7) et d'autre part par un corps à cames (17, 25) monté sur la sonde (7), bloqué en rotation par rapport à celle-ci et s'engageant dans le boudin à cames (16, 16', 16"),
**caractérisé en ce que**
le corps à cames est installé sur la section de sonde en boudin.

2. Dispositif à électrode selon la revendication 1, **caractérisé en ce que** le corps à cames est un anneau à cames (17) fermé, formé de préférence en seule pièce moulée, assise sur la section de sonde en boudin de la sonde (7), et s'engageant avec au moins une saillie à cames (18, 19) dans le boudin à cames (16).

3. Dispositif à électrode selon la revendication 1, **caractérisé en ce que** le corps à cames est un anneau de spirale (25) ouvert, assis sur la section de sonde en boudin de la sonde (17), avec le boudin à cames (16', 16") à pas correspondant, coulissant de façon coaxiale par translation dans le boudin à cames (16', 16"), tout en s'introduisant dans le boudin à cames (16', 16"), lors d'une rotation de la sonde (7).

4. Dispositif à électrode selon l'une des revendications précédentes, **caractérisé en ce que** le mouvement rotatif de la sonde (7) est délimité par des butées de rotation (21, 22, 23) définissant la position de fixation sortie maximale et la position passive enfoncée de l'électrode à vis (8) et coopérant avec le corps à cames (17, 25).

5. Dispositif à électrode selon la revendication 4, **caractérisé en ce que** les butées de rotation sont formées sur le corps d'électrode (1) par des saillies (23) passant dans la trajectoire de mouvement du corps à cames (25).

6. Dispositif à électrode selon la revendication 4, **caractérisé en ce que** les butées de rotation sont formées par les extrémités (21, 22) coudées vers l'intérieur dans la trajectoire de mouvement du corps à cames (17) du boudin à cames en forme de fil (16, 16").

7. Dispositif à électrode selon l'une des revendications précédentes, **caractérisé en ce que** la commande à cames (13, 13') est installée dans un boîtier d'isolation (14), radialement à l'intérieur d'une électrode annulaire (4) montée devant l'extrémité distale (3) de l'électrode.

8. Dispositif à électrode selon l'une des revendications 1 à 6, **caractérisé en ce que** la commande à cames (13") est installée dans un boîtier de tête (27) séparé.

9. Dispositif à électrode, en particulier selon l'une des revendications précédentes, **caractérisé en ce que** l'électrode à vis (8) est constituée d'un fil métallique avec une section transversale irrégulièrement ronde, dans lequel la dimension de section transversale (r) s'étendant radialement à l'axe de vissage (11) est supérieure à la dimension de section transversale (a) orientée parallèlement à celui-ci.

10. Dispositif à électrode selon la revendication 9, **caractérisé en ce que** la section transversale du fil présente une forme elliptique ou rectangulaire.
